# EUROPEAN PATENT APPLICATION

(11) **EP 1 870 477 A1**
(43) Date of publication of application: **26.12.2007**
(21) Application number: 06731119.1
(22) Date of filing: 29.03.2006
(51) Int. Cl.: C12Q 1/25, C12Q 1/48, C12Q 1/527, G01N 37/00

(54) **METHOD OF MEASURING ENZYME ACTIVITY AND METHOD OF EVALUATING COMPOUND**

(30) Priority: 01.04.2005 JP 2005106384
(71) Applicant: BANYU PHARMACEUTICAL CO., LTD., Tokyo 102-8667 (JP)
(72) Inventor: TOKITA, Shigeru Tsukuba Research Institute of, Tsukuba-shi Ibaraki 3002611 (JP); SHIMAMURA, Ken Tsukuba Research Institute of, Tsukuba-shi Ibaraki 3002611 (JP); MIYAMOTO, Yasuhisa Tsukuba Research Institute of, Tsukuba-shi Ibaraki 3002611 (JP); KITAZAWA, Hidefumi Tsukuba Research Institute of, Tsukuba-shi Ibaraki 3002611 (JP); KANATANI, Akio Tsukuba Research Institute of, Tsukuba-shi Ibaraki 3002611 (JP)
(74) Representative: Hussain, Deeba
(86) International application number: PCT/JP2006/307171
(87) International publication number: WO 2006/107063

(57) **Abstract**

It is intended to provide a method of measuring an activity, which is a method of measuring an activity of an enzymatic reaction in which a lipid-soluble reaction product is formed by catalyzing an addition reaction of two or more kinds of substrates, **characterized by** comprising a labeling step in which any one of the substrates is labeled, a reaction step in which an enzymatic reaction is carried out in the presence of an enzyme, all substrates and an acceptor, and a detection step in which the reaction product is detected by bringing the labeled molecule and a molecule to be detected close to each other via the acceptor and transferring an energy generated by the labeled molecule to the molecule to be detected, and a method of evaluating a compound utilizing the method of measuring an activity. By the methods of the invention, it is possible to perform a treatment from the enzymatic reaction to the measurement of an enzyme activity with the simple steps and it is possible to provide a method of measuring an enzyme activity which can be applied to an HTS evaluation system.

## Description

### Technical Field

The present invention relates to a method of measuring an activity of an enzymatic reaction in which a lipid-soluble reaction product is formed.

### Background Art

A pharmaceutical chemical is screened by evaluating a large number of compounds to become a candidate using a biochemical or cell biological evaluation system (for example, an enzymatic assay system or a binding assay system) based on a characteristic of an in vivo molecule to be a target for drug discovery. The probability of selecting one development candidate compound is said to be one in several tens of thousands of compounds, and it is essential for efficient pharmaceutical development that a large amount of development candidate compounds should be examined by improving the screening speed.

Under such circumstances, screening of a development candidate compound has been carried out by constructing an evaluation system called HTS (high through-put screening) in which screening efficiency is extremely accelerated and evaluating several tens of thousands to several hundreds of thousands of kinds of compounds in a short time.

However, it is necessary that the biochemical or cell biological evaluation system applicable to HTS should be constructed by a person who performs the evaluation. Therefore, it can be said that the completeness of such an evaluation system has a large influence on the efficient and effective screening.

On the other hand, in the case where a certain type of enzyme is set to be a target for drug discovery, a reaction product of the enzyme is a lipid-soluble substance in some cases. In the case where a reaction product was a lipid-soluble substance, when a quantitative or qualitative analysis thereof was carried out, it was necessary to perform an analysis by employing HPLC (high performance liquid chromatography), GC (gas chromatography), TLC (thin layer chromatography) and the like in combination after the reaction product was extracted with an organic solvent.

For example, JP-A-2003-212790 describes a method in which when a lipid-soluble component derived from Hericium erinaceum is extracted as a component effective in prevention and treatment of a neurodegenerative disease, the lipid-soluble component is extracted with acetone and chloroform and then the resulting extract is subjected to a silica gel column thereby to obtain a fraction.

Patent document 1: JP-A-2003-212790

However, in such a conventional method, it was necessary to undergo an extraction step with the use of an organic solvent when a lipid-soluble substance was extracted, and multiple steps from an enzymatic reaction to measurement of an enzyme activity were required. Therefore, there were problems that not only enormous labor and time were needed, but also the method could not be used in an HTS evaluation system in which a series of reactions, and treatment and measurement of reaction products were carried out in a microplate.

### Disclosure of the Invention

The present invention has been made in view of the above-mentioned problems of the conventional technique, and has its object to provide a method of measuring an enzyme activity which is capable of performing a treatment from an enzymatic reaction to measurement of an enzyme activity through a simple step and is applicable to an HTS evaluation system.

The present inventors made intensive studies in order to achieve the above object, and as a result, they found a method with which measurement of an enzyme activity can be performed without performing extraction of a reaction product by mixing a substance (acceptor) that binds to a lipid-soluble substance formed by an enzymatic reaction in an enzymatic reaction solution, and detecting the reaction product through the acceptor, and thus completed the present invention.

That is, the method of measuring an activity of the present invention is a method of measuring an activity of an enzymatic reaction in which a lipid-soluble reaction product is formed by catalyzing an addition reaction of two or more kinds of substrates, characterized by comprising: a labeling step in which any one of the substrates is labeled; a reaction step in which an enzymatic reaction is carried out in the presence of an enzyme, all substrates and an acceptor; and a detection step in which the reaction product is detected by bringing the labeled molecule and a molecule to be detected close to each other via the acceptor and transferring an energy generated by the labeled molecule to the molecule to be detected.

Further, the method of measuring an activity of the present invention is a method of measuring an activity of an enzymatic reaction in which a lipid-soluble reaction product is formed by catalyzing an addition reaction of two or more kinds of substrates, characterized by comprising: a labeling step in which any one of the substrates is labeled; a reaction step in which an enzymatic reaction is carried out in the presence of an enzyme and all substrates; a mixing step in which an acceptor that binds to the reaction product is mixed in the enzymatic reaction solution; and a detection step in which the reaction product is detected by bringing the labeled molecule and a molecule to be detected close to each other via the acceptor and transferring an energy generated by the labeled molecule to the molecule to be detected.

Here, in the method of measuring an activity of the present invention, the acceptor is preferably a substance that specifically binds to the reaction product. By using such an acceptor, higher accurate measurement of an enzyme activity becomes possible.

Further, the method of evaluating a compound of the present invention is a method of evaluating a compound that acts on an enzyme that catalyzes an addition reaction of two or more kinds of substrates, characterized by comprising: a labeling step in which any one of the substrates is labeled; a reaction step in which an enzymatic reaction is carried out in the presence of an enzyme, all substrates, an acceptor and a test compound; and a detection step in which the reaction product is detected by bringing the labeled molecule and a molecule to be detected close to each other via the acceptor and transferring an energy generated by the labeled molecule to the molecule to be detected.

Still further, the method of evaluating a compound of the present invention is a method of evaluating a compound that acts on an enzyme that catalyzes an addition reaction of two or more kinds of substrates, characterized by comprising: a labeling step in which any one of the substrates is labeled; a reaction step in which an enzymatic reaction is carried out in the presence of an enzyme, all substrates and a test compound; a mixing step in which an acceptor that binds to the reaction product is mixed in the enzymatic reaction solution; and a detection step in which the reaction product is detected by bringing the labeled molecule and a molecule to be detected close to each other via the acceptor and transferring an energy generated by the labeled molecule to the molecule to be detected.

Here, in the method of evaluating a compound of the present invention, the acceptor is preferably a substance that specifically binds to the reaction product. By using such an acceptor, higher accurate measurement of an enzyme activity becomes possible, and as a result, higher accurate evaluation of a compound becomes possible.

Further, a kit for measuring an enzyme activity of the present invention is characterized by comprising an enzyme that catalyzes a lipid-soluble reaction product, a substrate for the enzyme and an acceptor. By using such a kit, it becomes possible to conveniently carry out measurement of an activity of an enzymatic reaction in which a lipid-soluble substance is formed. In addition, it becomes possible to conveniently carry out evaluation and screening of a compound aiming at such an enzymatic reaction and the like.

It becomes possible to provide a method of measuring an enzyme activity which is capable of performing a treatment from an enzymatic reaction to measurement of an enzyme activity through a simple step and is applicable to an HTS evaluation system. Further, by utilizing the method of measuring an activity of the present invention, evaluation (measurement of an activity of a compound, screening or the like) of a compound that acts on an enzyme becomes possible.

### Brief Description of the Drawings

Fig. 1 is a view showing the results of measuring enzyme activities of LCE by a method of the present invention.
Fig. 2 is a view showing the results of measuring enzyme activities of hElovl-3 by a method of the present invention.
Fig. 3 is a view showing the results of measuring enzyme activities of hElovl-1 by a method of the present invention.

### Best Mode for Carrying Out the Invention

Hereinafter, a preferred embodiment of the present invention will be described in detail.

First, words and phrases to be used in the present invention will be described.

The "enzyme" according to the present invention is not particularly limited as long as it is an enzyme that catalyzes a reaction in which a lipid-soluble substance is formed as an enzymatic reaction product. Examples thereof include LCE (accession number: AK027031), FAS (fatty acid synthase) (accession number: BC063242), fatty acid elongases such as Elovl-1 (accession number: BC000618, NM_022821), Elovl-2 (accession number: BC060809), Elovl-3 (accession number: BC034344), Elovl-4 (accession number: AY037298) and Elovl-5 (accession number: AF338241), acyl-CoA synthases, GPAT (accession number: AL833061), DGAT1 (accession number: AF059202) or MGAT (accession number: AF384163), ACAT (accession number: L21934), and LCAT (accession number: NM_000229).

Further, as the enzyme according to the present invention, other than the enzymes listed above, for example, various membrane proteins, and various phospholipid or neutral lipid synthetic or metabolic enzymes are also exemplified as the enzyme according to the present invention. As such an enzyme, specifically, for example, acyltransferase, acetylhydrolase, lipase, glycerol kinase (accession number: NM_033214), choline phosphotransferase (accession number: NM_202244) can be exemplified. Further, membrane proteins of various cholesterol synthetic or metabolic enzymes and the like are also included in the enzyme according to the present invention.

Further, the "substrate" according to the present invention is not particularly limited as long as it is a substrate for the above-mentioned enzyme. However, because the enzyme catalyzes an addition reaction of two kinds of substrates, for example, in the case where the enzyme is LCE, two kinds of substrates: a long chain fatty acid (for example, palmitoyl-CoA), and a substrate that forms a longer chain fatty acid by addition to the long chain fatty acid (for example, malonyl-CoA) can be exemplified. Further, in the case of other enzymes, for example, a short chain or a medium chain fatty acid acyl-CoA and malonyl-CoA which are substrates for FAS, a saturated very long chain fatty acid acyl-CoA or an unsaturated fatty acid acyl-CoA which is a substrate for Elovls, a short chain or a long chain fatty acid and activated CoA which are substrates for an acyl-CoA synthase, a medium chain or a long chain fatty acid acyl-CoA and glycerol 3-phosphate which are substrates for GPAT, a medium chain or a long chain fatty acid acyl-CoA and glycerol 3-phosphate and lysophospholipid which are substrates for a phospholipid synthase, a medium chain or a long chain fatty acid acyl-CoA and diacylglycerol which are substrates for DGAT, and a medium chain or a long chain fatty acid acyl-CoA and monoacylglycerol which are substrates for MGAT can be exemplified. Further, in the case where the enzyme is glycerol kinase, glycerol and ATP are used as substrates, and in the case where the enzyme is choline phosphotransferase, diacylglycerol and CDP-choline are used as substrates.

Here, it is necessary that either one of the "substrates" according to the present invention should be modified such that it can be detected in the detection step. The mode of modification is not particularly limited and the substrate can be modified into a preferred form according to the detection method. For example, in the case of FRET (fluorescence resonance energy transfer), fluorescent dye labeling can be exemplified, and in the case of SPA (scintillation proximity assay), radioisotope labeling can be exemplified.

Further, the "lipid-soluble reaction product" according to the present invention is not particularly limited as long as it is a lipid-soluble substance formed by an enzymatic reaction of the above-mentioned enzyme. Examples thereof include acyl-CoA formed by an enzymatic reaction of LCE, a medium chain or a long chain fatty acid and a medium chain or a long chain fatty acid acyl-CoA formed by an enzymatic reaction of FAS, a saturated very long chain, a long chain fatty acid or an unsaturated fatty acid and a saturated very long chain, a long chain fatty acid or an unsaturated fatty acid acyl-CoA formed by an enzymatic reaction of Elvols, acyl-CoA formed by an enzymatic reaction of an acyl-CoA synthase, lyso-PA formed by an enzymatic reaction of GPAT, phospholipid formed by an enzymatic reaction of a phospholipid synthase, triacylglycerol formed by an enzymatic reaction of DGAT, and diacylglycerol formed by an enzymatic reaction of MGAT. Further, in the case where the enzyme is glycerol kinase, glycerol 3-phosphate and ADP are reaction products, and in the case where the enzyme is choline phosphotransferase, phosphatidylcholine is used as a substrate.

Further, the "acceptor" according to the present invention is not particularly limited as long as it is a substance that binds to the above-mentioned "lipid-soluble substance formed by an enzymatic reaction". Examples thereof include antibodies against the lipid-soluble substance, membrane receptors, nuclear receptors, and lipid transfer proteins having an affinity for the lipid-soluble substance, and the like. Further, the acceptor according to the present invention is preferably a substance that specifically binds to the lipid-soluble substance. Specific examples thereof include ACBP (acyl-CoA-binding protein) for acyl-CoA which is an enzymatic reaction product of LCE, lyso-PA- binding protein for lyso-PA which is an enzymatic reaction product of GPAT, and phospholipid-binding protein for phospholipid which is an enzymatic reaction product of PL synthesizing enzyme. Further, in the case of choline phosphotransferase, phosphatidylcholine transfer protein serves as an acceptor. By employing such an acceptor that shows specific binding, it becomes possible to perform accurate measurement of an enzyme activity without detecting noise due to nonspecific binding.

### (Method of measuring activity)

Subsequently, the method of measuring an activity of the present invention will be described.

The method of measuring an activity of the present invention is a method of measuring an activity of an enzymatic reaction in which a lipid-soluble reaction product is formed by catalyzing an addition reaction of two or more kinds of substrates, characterized by comprising: a labeling step in which any one of the substrates is labeled; a reaction step in which an enzymatic reaction is carried out in the presence of an enzyme, all substrates and an acceptor; and a detection step in which the reaction product is detected by bringing the labeled molecule and a molecule to be detected close to each other via the acceptor and transferring an energy generated by the labeled molecule to the molecule to be detected.

Hereinafter, the procedure of the method of measuring an activity of the present invention will be described.

In the method of measuring an activity of the present invention, first, a substrate is labeled such that it can be detected in the later detection step. The mode of labeling may be suitably selected according to the detection method, and for example, labeling with a radioisotope or a fluorescent dye can be exemplified. The labeling method is not particularly limited, and can be carried out by a known method (see, for example, Biochemistry, vol. 43, p. 2484, 2004).

Further, with regard to the substrate to be labeled, even in the case where there are two or more kinds of substrates, it is only necessary to label any one of the substrates.

By allowing the thus labeled substrate to coexist with an enzyme and other unlabeled substrates in the subsequent reaction step, an enzymatic reaction is allowed to proceed. Further, the reaction can be carried out by allowing an acceptor to coexist in this reaction solution. The conditions of the enzymatic reaction may be suitably set by considering the optimal temperature and optimal pH of the enzyme to be used, and the like. For example, conditions similar to those of living body in which the temperature is between 35 and 40°C and the pH is between 6.5 and 7.5 can be employed as preferred conditions. Further, with regard to a buffer solution in the reaction, a preferred buffer solution can be suitably used according to the enzyme to be used, and for example, a Tris-HCl buffer solution, a phosphate buffer solution, or an acetate buffer solution can be used. Further, these buffer solutions can be used by suitably adjusting various conditions such as a salt concentration.

The enzymatic reaction solution having undergone the reaction step subsequently proceeds to the mixing step. The mixing step is a step in which an acceptor is added and mixed following the enzymatic reaction in the case where the enzymatic reaction is carried out in the presence of only two or more kinds of substrates and an enzyme. In the case where the enzymatic reaction is carried out by allowing an acceptor to coexist in the enzymatic reaction solution in the reaction step, this step is not necessary. By the enzymatic reaction, a lipid-soluble reaction product is formed. Here, the addition and mixing method is not particularly limited, and for example, it can be carried out by preparing a solution containing an acceptor and adding the resulting solution to the enzymatic reaction solution, and then, stirring and mixing the resulting solution. Further, the mixing conditions are not particularly limited, and conditions suitable for binding between the reaction product and a substance to be bound thereto can be appropriately selected. By doing this, the acceptor binds to the reaction product, whereby a complex of the reaction product and the acceptor is formed.

Further, as described above, in the reaction step, the reaction can be carried out in a state in which an acceptor is also allowed to coexist in the enzymatic reaction solution containing an enzyme and a substrate for the enzyme. In this case, the acceptor sequentially binds to the reaction product resulted from the enzymatic reaction in the solution. Thus, it becomes possible to proceed to the detection step without undergoing the mixing step.

In the complex formed in the mixing step, a label derived from the substrate in a detectable form is contained. Therefore, in the subsequent detection step, by detecting such a label, the presence of the reaction product is to be confirmed. Because the reaction product is labeled with a fluorescent dye, a radioisotope or the like derived from the substrate, a preferred detection method can be suitably adopted according to the type of label in the detection step. To be more specific, for example, in the case where the label is a fluorescent dye, the label can be detected using a medium or an apparatus (for example, an FRET system or a Top Count) that detects a fluorescence. Further, in the case where the label is a radioisotope, the label can be detected using a medium or an apparatus (for example, an SPA system, a scintillation counter or a CCD camera) that detects a radioisotope.

Further, by constructing a calibration curve using various standard enzyme products separately prepared by synthesis or purification, specifically, for example, various standard enzyme products labeled with a fluorescent dye, a radioisotope or the like and various acceptors, it becomes possible to determine an activity of an enzyme contained in the assay system from a value detected in the above-mentioned assay.

According to the method of measuring an activity of the present invention as described in the above, an enzyme activity can be measured without subjecting a lipid-soluble reaction product formed by an enzymatic reaction to a step of extraction with an organic solvent or the like. Since the extraction with an organic solvent requires multiple steps of mixing, stirring and extraction, by performing the extraction of a reaction product without undergoing such steps, it becomes possible to perform prompt extraction by a very simple step, and as a result, it becomes possible to perform measurement or evaluation of activities of a large number of test substances in a short time. Further, in the extraction with an organic solvent, a solvent harmful to environment and the human body is used in many cases, and also labor and cost are required for the treatment of waste liquid. Therefore, the advantage of being able to measure an activity without using an organic solvent is very large.

### (Method of evaluating compound)

Subsequently, the method of evaluating a compound of the present invention will be described.

The method of evaluating a compound of the present invention is a method of evaluating a compound that acts on an enzyme that catalyzes an addition reaction of two or more kinds of substrates, characterized by comprising: a labeling step in which any one of the substrates is labeled; a reaction step in which an enzymatic reaction is carried out in the presence of an enzyme, all substrates, an acceptor and a test compound; and a detection step in which the reaction product is detected by bringing the labeled molecule and a molecule to be detected close to each other via the acceptor and transferring an energy generated by the labeled molecule to the molecule to be detected.

Here, the "test compound" according to the present invention is not particularly limited in terms of its type as long as it is a compound targeting the "enzyme" according to the present invention. As the compound targeting the enzyme, for example, agonists, antagonists and inverse agonists of the enzyme can be exemplified. Further, the mode of binding between the enzyme and the test compound is not particularly limited, and for example, in the case of an inhibitory reaction, it may be either competitive inhibition or non-competitive inhibition.

The method of evaluating a compound of the present invention is carried out by allowing further a test compound to coexist in the reaction step of the above-mentioned method of measuring an activity of the present invention and allowing an enzymatic reaction to proceed in such a condition. By doing this, the test compound can be evaluated by detecting in the detection step, a change in the activity of an enzymatic reaction caused by the test compound when the test compound has a some kind of effect on the enzyme and as a result, the enzymatic reaction is inhibited or promoted.

Hereinafter, the procedure of the method of evaluating a compound of the present invention will be described.

In the method of evaluating a compound of the present invention, first, a substrate is labeled such that it can be detected in the later detection step. The mode of labeling may be suitably selected according to the detection method, and for example, labeling with a radioisotope or a fluorescent dye can be exemplified. The labeling method is not particularly limited, and can be carried out by a known method (see, for example, Biochemistry, vol. 43, p. 2484, 2004).

Further, with regard to the substrate to be labeled, it is only necessary to label any one of the two or more kinds of substrates.

By allowing the thus labeled substrate to coexist with an enzyme, other unlabeled substrates and a test compound in the subsequent reaction step, an enzymatic reaction is allowed to proceed. Further, the reaction can be carried out by allowing an acceptor to coexist in this reaction solution. The conditions of the enzymatic reaction may be suitably set by considering the optimal temperature and optimal pH of the enzyme to be used, and the like. For example, conditions similar to those of living body in which the temperature is between 35 and 40°C and the pH is between 6.5 and 7.5 can be employed as preferred conditions. Further, with regard to a buffer solution in the reaction, a preferred buffer solution can be suitably used according to the enzyme to be used, and for example, a Tris-HCl buffer solution, a phosphate buffer solution, or an acetate buffer solution can be used. Further, these buffer solutions can be used by suitably adjusting a salt concentration or the like.

Subsequently, the enzymatic reaction solution having undergone the reaction step proceeds to the mixing step. The mixing step is a step in which an acceptor is added and mixed following the enzymatic reaction in the case where the enzymatic reaction is carried out in the presence of only two or more kinds of substrates and an enzyme. In the case where the enzymatic reaction is carried out by allowing an acceptor to coexist in the enzymatic reaction solution in the reaction step, this step is not necessary. By the enzymatic reaction, a lipid-soluble reaction product is formed. Here, the addition and mixing method is not particularly limited, and for example, it can be carried out by preparing a solution containing an acceptor and adding the resulting solution to the enzymatic reaction solution, and then, stirring and mixing the resulting solution. Further, the mixing conditions are not particularly limited, and conditions suitable for binding between the reaction product and a substance to be bound thereto can be appropriately selected. By doing this, the acceptor binds to the reaction product, whereby a complex is formed.

Further, in the reaction step, the reaction can be carried out in a state in which an acceptor is also allowed to coexist in the enzymatic reaction solution containing a test compound, an enzyme and a substrate for the enzyme. In this case, the acceptor sequentially binds to the reaction product resulted from the enzymatic reaction in the solution. Thus, it becomes possible to proceed to the detection step without undergoing the mixing step.

In the complex formed in the mixing step, a label derived from the substrate in a detectable form is contained. Therefore, in the subsequent detection step, by detecting such a label, the presence of the reaction product is to be confirmed. Because the reaction product is labeled with a fluorescent dye, a radioisotope or the like derived from the substrate, a preferred detection method can be suitably adopted according to the type of label in the detection step. To be more specific, for example, in the case where the label is a fluorescent dye, the label can be detected using a medium or an apparatus (for example, an FRET system or a Top Count) that detects a fluorescence. Further, in the case where the label is a radioisotope, the label can be detected using a medium or an apparatus (for example, an SPA system, a scintillation counter or a CCD camera) that detects a radioisotope.

Further, by constructing a calibration curve using various standard enzyme products separately prepared by synthesis or purification, specifically, for example, various standard enzyme products labeled with a fluorescent dye, a radioisotope or the like and various acceptors, it becomes possible to determine an activity of an enzyme contained in the assay system from a value detected in the above-mentioned assay.

As a specific example of the evaluation of a test compound, for example, in the case where the evaluation of plural types of test compounds are carried out using LCE as the enzyme, when the enzyme activity of LCE is inhibited in the presence of a certain test compound and as a result, the production amount of a lipid-soluble substance (for example, acyl-CoA) formed by the enzymatic reaction is reduced compared with a control group (a condition in which the test compound is not allowed to coexist), the test compound is recognized to function as an inhibitor of LCE. LCE has a function to promote fatty acid synthesis by elongating the carbon chain of fatty acid, therefore, by inhibiting this action, the synthesis of fatty acid is inhibited, resulting in inhibiting the synthesis of lipid. That is, it can be considered that the test compound is a compound exhibiting an anti-obesity effect.

According to the method of evaluating a compound of the present invention as described in the above, an enzyme activity can be measured without subjecting a lipid-soluble reaction product formed by an enzymatic reaction to a step of extraction with an organic solvent or the like, therefore, it becomes possible to evaluate a test compound that has an effect of promoting or inhibiting the reaction by acting on the enzyme. Since the extraction with an organic solvent requires multiple steps of mixing, stirring and extraction, by performing the extraction of a reaction product without undergoing such steps, it becomes possible to perform prompt extraction by a very simple step, and as a result, it becomes possible to perform measurement or evaluation of activities of a large number of test compounds in a short time.

Further, in the extraction with an organic solvent, a solvent harmful to environment and the human body is used in many cases, and also labor and cost are required for the treatment of waste liquid. Therefore, the advantage of the method of evaluating a compound of the present invention with which the measurement of an activity can be carried out without using an organic solvent is very large.

### (Examples)

Hereinafter, the present invention will be described more specifically with reference to Examples, however, the invention is not limited to the following Examples.

### Example 1

### (Preparation of a human long chain fatty acid CoA-binding protein tagged with a His (histidine) tag at the N terminus)

The cDNA sequence encoding human ACBP protein (Biochimica et biophysica acta, vol. 1441, p. 150, 1999) was amplified by RT-PCR using human liver mRNA (manufactured by Clontech) as a template.

Subsequently, the amplified product was cloned into an expression vector tagged with a His tag, pET-14b (manufactured by Novagen), whereby an expression vector that expresses a human long chain fatty acid CoA-binding protein tagged with a His tag at the N terminus (hereinafter referred to as N-Tag-hACBP) was constructed. The resulting expression vector was transfected into a host E. coli by the calcium chloride method, whereby a transformant was obtained.

By culturing the resulting transformant in a medium supplemented with isopropyl-beta-D-thiogalactoside, the expression of N-Tag-hACBP was induced, and N-Tag-hACBP was purified using His-Bind kits (manufactured by Novagen) from a bacterial cytosolic fraction. The purified N-Tag-hACBP was used in the following experiment.

### (Preparation of hLCE (human long chain fatty acyl elongase))

The cDNA sequence encoding hLCE (Journal of biological chemistry, vol. 276, p. 45358, 2001, accession number: AK027031) was amplified by RT-PCR using human liver mRNA (manufactured by Clontech), and then the amplified product was cloned into an expression vector, pPICZα (manufactured by Invitrogen).

The resulting expression vector was transfected into a host yeast, Pichia pastris (manufactured by Invitrogen) by the electroporation method, and from the transformed yeast, a microsomal sample was obtained by a standard method.

### (Method of measuring LCE activity using N-Tag-hACBP)

An assay buffer solution (100 mM sodium chloride, 100 mM Tris buffer solution, pH 6.5) containing the above-mentioned yeast microsomal sample, 1 µM rotenone (manufactured by Sigma), 1 mM NADPH (reduced nicotinamide adenine dinucleotide phosphate, manufactured by Sigma), 20 µM fatty acid-free bovine serum albumin (manufactured by Sigma), 40 µM long chain fatty acid CoA (manufactured by Sigma), and 50,000 dpm of 60 µM malonyl-CoA (manufactured by Sigma, Muromachi Yakuhin Kagaku) was incubated at 37°C for 2 hours in a 96-well Opti-plate (Packard), and then, 0.25% octyl-beta-glucoside was added thereto to stop the reaction.

Subsequently, 2 µg of a human acyl-CoA-binding protein tagged with a His tag at the N terminus and 500 µg of SPA resin (PVT copper His-Tag SPA Beads, manufactured by Amersham) were added thereto, and the resulting mixture was incubated at 37°C for 30 minutes.

The solution after incubation was centrifuged at 3,000 rpm, and an activity was measured using a Top Count (Packard). Here, in order to evaluate a specific activity, an expression vector in which hLCE was not cloned was transfected into a host yeast, Pichia pastris by the electroporation method, whereby a transformed yeast was obtained. From the resulting transformed yeast, a microsomal sample was obtained according to a standard method, and comparison was carried out with the activity of the microsomal sample derived from the transformed yeast in which N-Tag-hACBP was introduced.

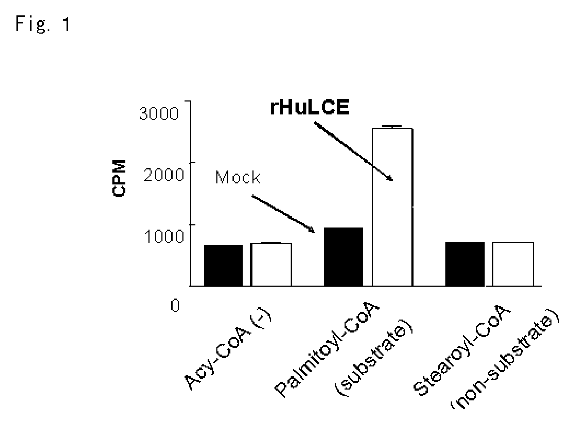

As shown in Fig. 1, specific enhancement of a signal was observed only in the case where palmitoyl-CoA was used as the substrate and N-Tag-hACBP microsomal sample was added to the reaction, and enhancement of a signal was not observed in the case where stearyl-CoA which is a non-substrate was used as the substrate, or the case where the microsomal sample prepared from the hLCE non-expressing strain was used as the enzyme. In Fig. 1, rHuLCE indicates recombinant human LCE, and Mock indicates a control.

From the above results, it could be confirmed that hLCE-specific activity can be measured by the method of the present invention.

### Examples 2 and 3

### (Method of preparing hElovl-3 (human elongase of very long chain fatty acids-like 3))

In the same manner as the method of measuring an activity of LCE described in Example 1, measurement of an activity of hElovl-3 was attempted.

First, the cDNA sequence encoding hElovl-3 (accession number: BC034344) was amplified by RT-PCR using human liver mRNA (manufactured by Clontech), and then the amplified product was cloned into an expression vector, pCMV-Tag2B (manufactured by Stratagene). The resulting expression vector was transfected into a host cell, HEK293 using the host cationic lipid method (Proc. Natl. Acad. Sci. U.S.A., vol. 84, p. 7413, 1987), and from the expressing cell, a microsomal sample was obtained by a standard method.

### (Method of preparing hElovl-1 (human elongase of very long chain fatty acids-like 1))

The cDNA sequence encoding hElovl-1 (accession number: NM_022821) was amplified by RT-PCR using human liver mRNA (manufactured by Clontech), and then the amplified product was cloned into an expression vector, pCMV-Tag2B (manufactured by Stratagene). The resulting expression vector was transfected into a host cell, HEK293 using the host cationic lipid method (Proc. Natl. Acad. Sci. U.S.A., vol. 84, p. 7413, 1987), and from the expressing cell, a microsomal sample was obtained by a standard method.

### (Method of measuring activities of Elovl-3 (Example 2) and hElovl-1 (Example 3) using N-Tag-hACBP)

An assay buffer solution (100 mM sodium chloride, 100 mM Tris buffer solution, pH 6.5) containing the cell microsomal sample of hElovl-3 or hElovl-1 obtained by the above-mentioned preparation method, 1 µM rotenone (manufactured by Sigma), 1 mM NADPH (reduced nicotinamide adenine dinucleotide phosphate, manufactured by Sigma), 20 µM fatty acid-free bovine serum albumin (manufactured by Sigma), 40 µM long chain fatty acid CoA (manufactured by Sigma, manufactured by Avanti), and 50,000 dpm of 60 µM malonyl-CoA (manufactured by Sigma, Muromachi Yakuhin Kagaku) was incubated at 37°C for 2 hours in a 96-well Opti-plate (Packard), and then, 0.25% octyl-beta- glucoside was added thereto to stop the reaction.

Subsequently, 2 µg of a human acyl-CoA-binding protein tagged with a His tag at the N terminus and 500 µg of SPA resin (PVT copper His-Tag SPA Beads, manufactured by Amersham) were added to the reaction solution, and the resulting mixture was incubated at 37°C for 30 minutes, followed by centrifugation at 3,000 rpm, and an activity was measured using a Top Count (Packard).

In order to evaluate a specific activity, an expression vector in which either enzyme was not cloned was transfected into a host cell, HEK293 by the host cationic lipid method, and comparison was carried out with the microsomal sample obtained by a standard method from the expressing cell.

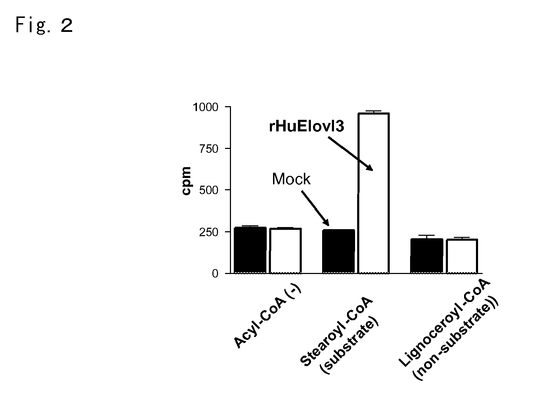

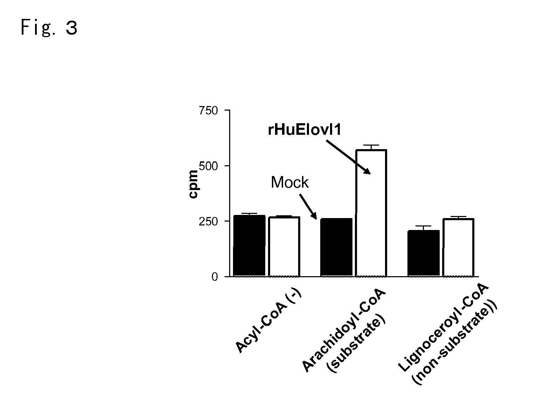

As a result, in the case of hElovl-3, specific enhancement of a signal was observed only in the case where stearoyl-CoA was used as the substrate and N-Tag-hACBP (added to the reaction solution as the microsomal sample) was used as the acceptor (Fig. 2). Further, in the case of hElovl-1, specific enhancement of a signal was observed only in the case where arachidoyl-CoA was used as the substrate and N-Tag-hACBP (added to the reaction solution as the microsomal sample) was used as the acceptor (Fig. 3). On the other hand, in either of the cases of hElovl-3 and hElovl-1, enhancement of a signal was not observed in the case where lignoceroyl-CoA which is a non-substrate was used as the substrate, or the case where the microsome prepared from the hElovl-3 or hElovl-1 non-expressing cell was used as the enzyme. From these results, it could be confirmed that an activity specific to hElovl-3 and hElovl-1 can be measured by the method of measuring an enzyme activity of the present invention.

### Industrial Applicability

It becomes possible to provide a method of measuring an enzyme activity which is capable of performing a treatment from an enzymatic reaction to measurement of an enzyme activity through a simple step and is applicable to an HTS evaluation system. As a result, evaluation of a test compound can be simply and promptly carried out.

## Claims

1. A method of measuring an activity, which is a method of measuring an activity of an enzymatic reaction in which a lipid-soluble reaction product is formed by catalyzing an addition reaction of two or more kinds of substrates, **characterized by** comprising:
a labeling step in which any one of the substrates is labeled;
a reaction step in which an enzymatic reaction is carried out in the presence of an enzyme, all substrates and an acceptor; and
a detection step in which the reaction product is detected by bringing the labeled molecule and a molecule to be detected close to each other via the acceptor and transferring an energy generated by the labeled molecule to the molecule to be detected.

2. A method of measuring an activity, which is a method of measuring an activity of an enzymatic reaction in which a lipid-soluble reaction product is formed by catalyzing an addition reaction of two or more kinds of substrates, **characterized by** comprising:
a labeling step in which any one of the substrates is labeled;
a reaction step in which an enzymatic reaction is carried out in the presence of an enzyme and all substrates;
a mixing step in which an acceptor that binds to the reaction product is mixed in the enzymatic reaction solution; and
a detection step in which the reaction product is detected by bringing the labeled molecule and a molecule to be detected close to each other via the acceptor and transferring an energy generated by the labeled molecule to the molecule to be detected.

3. The method of measuring an activity according to claim 1 or 2, **characterized in that** the enzyme is LCE (a long chain fatty acid elongase).

4. The method of measuring an activity according to any one of claims 1 to 3, **characterized in that** the acceptor is a substance that specifically binds to the reaction product.

5. The method of measuring an activity according to any one of claims 1 to 4, **characterized in that** the detection means in the detection step is SPA.

6. A method of evaluating a compound, which is a method of evaluating a compound that acts on an enzyme that catalyzes an addition reaction of two or more kinds of substrates, **characterized by** comprising:
a labeling step in which any one of the substrates is labeled;
a reaction step in which an enzymatic reaction is carried out in the presence of an enzyme, all substrates, an acceptor and a test compound; and
a detection step in which the reaction product is detected by bringing the labeled molecule and a molecule to be detected close to each other via the acceptor and transferring an energy generated by the labeled molecule to the molecule to be detected.

7. A method of evaluating a compound, which is a method of evaluating a compound that acts on an enzyme that catalyzes an addition reaction of two or more kinds of substrates, **characterized by** comprising:
a labeling step in which any one of the substrates is labeled;
a reaction step in which an enzymatic reaction is carried out in the presence of an enzyme, all substrates and a test compound;
a mixing step in which an acceptor that binds to the reaction product is mixed in the enzymatic reaction solution; and
a detection step in which the reaction product is detected by bringing the labeled molecule and a molecule to be detected close to each other via the acceptor and transferring an energy generated by the labeled molecule to the molecule to be detected.

8. The method of evaluating a compound according to claim 6 or 7, **characterized in that** the enzyme is LCE (a long chain fatty acid elongase).

9. The method of evaluating a compound according to any one of claims 6 to 8, **characterized in that** the acceptor is a substance that specifically binds to the reaction product.

10. The method of evaluating a compound according to any one of claims 6 to 9, **characterized in that** the detection means in the detection step is SPA.

11. A kit for measuring an enzyme activity, **characterized by** comprising an enzyme that catalyzes a lipid-soluble reaction product, a substrate for the enzyme and an acceptor.
